# EUROPEAN PATENT SPECIFICATION

(11) **EP 4 338 712 B1**
(45) Date of publication and mention of the grant of the patent: **04.11.2026**
(21) Application number: 22827383.5
(22) Date of filing: 08.06.2022
(51) Int. Cl.: A61F 2/82, A61F 2/86, A61F 2/89, A61M 29/00, A61M 29/02

(54) **STENT AND MEDICINE CARRYING STENT**
STENT UND ARZNEIMITTELTRAGENDER STENT
STENT ET STENT DE TRANSPORT DE MÉDICAMENT

(30) Priority: 22.06.2021 CN 202110693992
(43) Date of publication of application: 20.03.2024
(73) Proprietor: MicroPort NeuroTech (Shanghai) Co., Ltd., Shanghai 201318 (CN)
(72) Inventor: SONG, Zhihao, Shanghai 201318 (CN); TIAN, Hao, Shanghai 201318 (CN); HOU, Juan, Shanghai 201318 (CN); LEI, Wenbin, Shanghai 201318 (CN)
(74) Representative: Marks & Clerk LLP
(86) International application number: PCT/CN2022/097716
(87) International publication number: WO 2022/267887

(56) References cited:
- EP-A1- 2 210 573
- CN-A- 101 411 651
- CN-A- 102 525 701
- CN-A- 107 049 571
- CN-A- 107 242 922
- CN-C- 100 471 470
- US-A1- 2003 105 511
- US-A1- 2004 225 350
- US-A1- 2004 249 443
- US-A1- 2010 161 027
- US-A1- 2012 029 620

## Description

### TECHNICAL FIELD

The present invention pertains to the field of medical devices. In particular, it relates to a stent and a drug-loaded stent.

### BACKGROUND

Intracranial atherosclerotic stenosis (ICAS) is a significant cause of the occurrence, development and recurrence of ischemic stroke. Clinical intervention focuses on ICAS cases with symptoms, and principal therapeutic measures include antiplatelet medication, angioplasty and traditional surgery. Although antiplatelet medication is a classic therapy for ICAS, it is associated with a high incidence of stroke. Traditional surgery is highly risky and therefore seldom employed.

As a result of developments in intracranial intervention, currently, angioplasty has become an important method for symptomatic ICAS treatment. Angioplasty procedures include dilation with a bare metal stent, a simple balloon, a coronary drug-coated stent or the like. However, bare metal stent or simple balloon based dilation is associated with a risk of restenosis, and coronary drug-coated stents are substantially balloon-expanded drug-loaded stents. Such stents have a great outer diameter dimension when loaded in a delivery system, which makes delivery to a target site difficult, in particular in a tortuous blood vessel or complex lesion. In contrast, self-expanding stents can be more easily delivered to a target site because of a reduced outer diameter dimension when loaded in a delivery system.

Differing from extracranial arteries, the physiological structure of an intracranial artery is characterized by: 1) great physiological curvature, as well as significant physiological tortuosity, in particular when it is arteriosclerotic usually as a consequence of stenosis; 2) a thin and inelastic outer layer, which is located in the subarachnoid space without support from tissue and therefore exhibits poor resistance to mechanical damage. When a drug escapes from an intracranial artery through a rupture in its wall, or permeates therethrough, it tends to spread through the cerebrospinal fluid across the entire brain and even reach the spinal cord. Accordingly, stents for use in intracranial arteries are desired to have suitable radial strength, which allows easy delivery to a target site and prevent damage from being caused to any blood vessel during use. On the other hand, in order to be able to compress a plaque in a desired manner, a stent is required to have sufficient metal coverage. However, for an intracranial blood vessel, which is vulnerable and lacks elasticity, high metal coverage causes an excessive radial strength, which may lead to damage to the intracranial blood vessel and hence possible in-stent restenosis.

Thus, conventional stents fail to provide good sufficient metal coverage and desirable radial strength.

EP2210573A1 discloses a nested stent in a body lumen, which is formed with a plurality of stent struts connected each other, the adjacent stent struts are nested each other when the stent is pressed and held, the said stent struts are provided with some interlace-arranged segments, the segments on the adjacent stent struts are formed mesh shape and nested each other. CN100471470C discloses a microporous blood vessel stent for eluting medicaments with a microcellular structure that improves drug loading and designed to enable controlled drug release. US2004225350A1 discloses an expandable medical device having a plurality of elongated struts, the plurality of elongated struts being joined together to form a substantially cylindrical device which is expandable. At least one of the plurality of struts includes at least one opening where a beneficial agent may be loaded into the opening within the strut.

### SUMMARY OF THE INVENTION

It is an object of the present invention to provide a stent, which can overcome the problem of failing to provide both good sufficient metal coverage and desirable radial strength associated with conventional stents.

To this end, a first aspect of the present invention provides a stent which comprises at least one stent mesh each comprising a plurality of stent struts sequentially connected circumferentially around the stent mesh, the plurality of the stent struts sequentially connected end to end, a joint formed at the connected ends of adjacent stent struts, each stent mesh configured to expand or collapse as a result of widening or narrowing of angles at the joints,
wherein each stent strut comprises at least one main section and at least one broadened section that are alternately arranged, the main section having a width smaller than a width of the broadened section; in each stent mesh, the broadened sections of adjacent stent struts are staggered along an axis of the stent mesh; in at least one of the stent struts, a total length of the at least one broadened section accounts for 5% to 95% of a length of the stent strut; and when the angle at each joint is in the range of 0° to 5°, a metal coverage of the stent mesh ranges from 30% to 99%. When the angle at each joint is in the range of 5° to 30°, the metal coverage of the stent mesh may range from 5% to 90%. In at least one of the stent struts, the broadened section may be flush with the main section at one side of the stent strut along a lengthwise direction thereof. In an expanded configuration of the stent, the adjacent stent struts connected to the same joint may form a V-shaped structure, wherein the sides of the adjacent stent struts, at which the main sections are flush with the broadened sections, are simultaneously located at inner side or outer side of the V-shaped structure.

Optionally, in at least one of the stent struts, a total length of the at least one broadened section may account for 15% to 75% of a length of the stent strut.

Optionally, in at least one of the stent struts, a total length of the at least one broadened section may account for 20% to 50% of a length of the stent strut.

Optionally, in adjacent two stent struts, a ratio of a total length of the broadened sections of these two stent struts to a length of the single stent strut may not exceed 100%.

Optionally, the angle at each joint may range from 0° to 140°.

Optionally, when the angle at each joint is in the range of 30° to 90°, the metal coverage of the stent mesh may range from 4% to 15%.

Optionally, when the angle at each joint is in the range of 30° to 90°, the metal coverage of the stent mesh may range from 8% to 15%.

Optionally, when the angle at each joint is in the range of 90° to 140°, the metal coverage of the stent mesh may range from 3% to 12%.

Optionally, when the angle at each joint is in the range of 0° to 5°, the metal coverage of the stent may range from 20% to 60%.

Optionally, when the angle at each joint is in the range of 5° to 30°, the metal coverage of the stent may range from 5% to 45%.

Optionally, when the angle at each joint is in the range of 30° to 90°, the metal coverage of the stent may range from 3% to 15%.

Optionally, when the angle at each joint is in the range of 90° to 140°, the metal coverage of the stent may range from 2% to 15%.

Optionally, the stent may have radial strength in the range of 1 kPa to 300 kPa.

Optionally, at least one of stent strut may comprise two main sections and one broadened section that is located between the two main sections.

Optionally, at least one of stent mesh may comprise 8 to 24 stent struts.

Optionally, at least one of the broadened section may comprise a cavity.

Optionally, the broadened section may comprise 1 to 10 cavities.

Optionally, a shape of the cavity may comprise at least one of a cylinder, a cuboid and a triangular prism.

Optionally, a transverse cross-sectional shape of the cavity may comprise at least one of a circular shape, an elongate shape, a polygonal shape, a corrugated shape, an annular shape and an irregular shape.

Optionally, the cavity may be configured for a drug or radiopaque substance to be filled therein.

Optionally, when the angles at the joints are minimized, in each adjacent stent struts connected to the same joint: the broadened section of one of the stent struts may not overlap with the main section of the adjacent stent strut.

Optionally, the stent may comprise at least two stent meshes that are axially connected.

Optionally, the stent may comprise linking struts, which connect the joints in adjacent stent meshes.

Optionally, a shape of the linking struts may comprise at least one of a linear shape, a corrugated shape, a serrated shape, a circular shape, an annular shape, a "Ω"-like shape and an "S"-like shape.

To the above end, a second aspect of the present invention provides a drug-loaded stent comprising a stent according to the first aspect of the present invention, wherein the stent comprises at least one stent mesh each comprising a plurality of stent struts sequentially connected circumferentially around the stent mesh, the plurality of the stent struts sequentially connected end to end, a joint formed at the connected ends of adjacent stent struts, the stent mesh configured to expand or collapse as a result of widening or narrowing of angles at the joints,
wherein each stent strut comprises at least one main section and at least one broadened section that are alternately arranged, the main section having a width smaller than a width of the broadened section; in each stent mesh, the broadened sections of adjacent stent struts are staggered along an axis of the stent mesh; the at least one broadened section comprises a cavity configured for a drug or radiopaque substance to be filled therein;
in at least one stent strut, a total length of the at least one broadened section accounts for 5% to 95% of a length of the stent strut; and when the angle at each joint is in the range of 0° to 5°, metal coverage of the stent mesh ranges from 30% to 99%.

The stent provided herein includes at least one stent mesh each including a plurality of stent struts, which are connected end to end circumferentially around the stent mesh and a joint is formed at the connected ends of adjacent stent struts. The stent mesh is configured to expand or collapse as a result of widening or narrowing of angles at the joints. Each stent strut includes main sections and broadened sections that are alternately arranged. The main sections have a width smaller than that of the broadened sections. In each stent mesh, the broadened sections of adjacent stent struts are staggered along an axis of the stent mesh. The drug-loaded stent provided herein is of a similar structure. The different widths of the broadened sections and the main sections, as well as the staggered arrangement of the broadened sections, impart to the present invention at least one of the following advantages over the prior art.
1. Reasonable space utilization and a good tradeoff between metal coverage and radial strength are achieved, overcoming the problem of failing to provide both good sufficient metal coverage and desirable radial strength associated with conventional stents.
2. The staggered arrangement of the broadened sections allows the stent to have a compact size when in a collapsed configuration, which facilitates passage of the stent through a lesion site.
3. Since the broadened sections have a relatively large surface area, cavities can be formed therein, in which a drug or radiopaque substance can be filled to increase the stent's drug loading capacity or radiopacity.

### BRIEF DESCRIPTION OF THE DRAWINGS

Those of ordinary skill in the art would appreciate that the accompanying drawings are provided to facilitate a better understanding of the present invention and do not limit the scope thereof in any sense, in which:
Fig. 1a is a schematic partial view of a stent mesh according to an embodiment of the present invention;
Fig. 1b is a schematic diagram showing the stent mesh of Fig. 1a in a collapsed configuration;
Fig. 2 is a schematic partial view of a stent mesh according to another embodiment of the present invention;
Fig. 3a is a schematic partial view of a stent mesh according to yet another embodiment of the present invention;
Fig. 3b is a schematic diagram showing the stent mesh of Fig. 3a in a collapsed configuration;
Fig. 4a is a schematic diagram showing a broadened section according to an embodiment of the present invention;
Fig. 4b is a schematic diagram showing a broadened section according to another embodiment of the present invention;
Fig. 4c is a schematic diagram showing a broadened section according to yet another embodiment of the present invention;
Fig. 5a schematically illustrates a first optional implementation of a cavity pattern according to the present invention;
Fig. 5b schematically illustrates a second optional implementation of a cavity pattern according to the present invention;
Fig. 5c schematically illustrates a third optional implementation of a cavity pattern according to the present invention;
Fig. 5d schematically illustrates a fourth optional implementation of a cavity pattern according to the present invention;
Fig. 5e schematically illustrates a fifth optional implementation of a cavity pattern according to the present invention;
Fig. 6 is a schematic partial view of a stent according to an embodiment of the present invention;
Fig. 7a is a schematic partial view of a stent mesh according to the present invention, particularly showing an implementation of joints therein;
Fig. 7b is a schematic diagram showing the stent mesh of Fig. 7a in a collapsed configuration;
Fig. 8a is a schematic partial view of a stent mesh according to the present invention, particularly showing another implementation of joints therein;
Fig. 8b is a schematic diagram showing the stent mesh of Fig. 8a in a collapsed configuration;
Fig. 9a is a schematic partial view of a stent mesh according to the present invention, particularly showing yet another implementation of joints therein; and
Fig. 9b is a schematic diagram showing the stent mesh of Fig. 9a in a collapsed configuration.

In these figures,
100, a stent mesh; 110, a stent strut; 120, a joint; 130, a linking strut; 101, a main section; 102, a broadened section; 103, a main segment; 104, a transition segment; 105, a cavity; 121, a bend section; and 200, a widthwise direction.

### DETAILED DESCRIPTION

Objects, advantages and features of the present invention will become more apparent upon reading the following more detailed description of the present invention with reference to the accompanying drawings. Note that the figures are provided in a very simplified form not necessarily drawn to exact scale and for the only purpose of facilitating easy and clear description of the embodiments. In addition, the structures shown in the figures are usually part of actual structures. In particular, as the figures tend to have distinct emphases, they are often drawn to different scales.

As used herein, the singular forms "a", "an" and "the" include plural referents. Herein, the term "or" is generally employed in the sense of "and/or", "several" is generally employed in the sense of "at least one", and "at least two" is generally employed in the sense of "two or more". Additionally, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two of the items. As used herein, the term "proximal end" generally refers to an end closer to an operator, and the term "distal end" generally refers to an end closer to a patient. The terms "one end" and "the other end", as well as "proximal end" and "distal end", are generally used to refer to opposite end portions including the opposite endpoints, rather than only to the endpoints. The terms "mounting", "coupling" and "connection" should be interpreted in a broad sense. For example, a connection may be a permanent, detachable or integral connection, a mechanical connection or an electrical connection, a direct or indirect connection with one or more intervening media, or an internal communication or interaction between two elements. As used herein, when an element is referred to as being "disposed on" another element, this is generally intended to only mean that there is a connection, coupling, engagement or transmission relationship between the two elements, which may be either direct or indirect with one or more intervening elements, and should not be interpreted as indicating or implying a particular spatial position relationship between the two elements, i.e., the element may be located inside, outside, above, under, beside, or at any other location with respect to the other element, unless the context clearly dictates otherwise. Those of ordinary skill in the art can understand the specific meanings of the above-mentioned terms herein, depending on their context.

In principle, the present invention seeks to provide a stent, which can overcome the problem of failing to provide good sufficient metal coverage and desirable radial strength associated with conventional stents.

The invention will be described below with reference to the accompanying drawings.

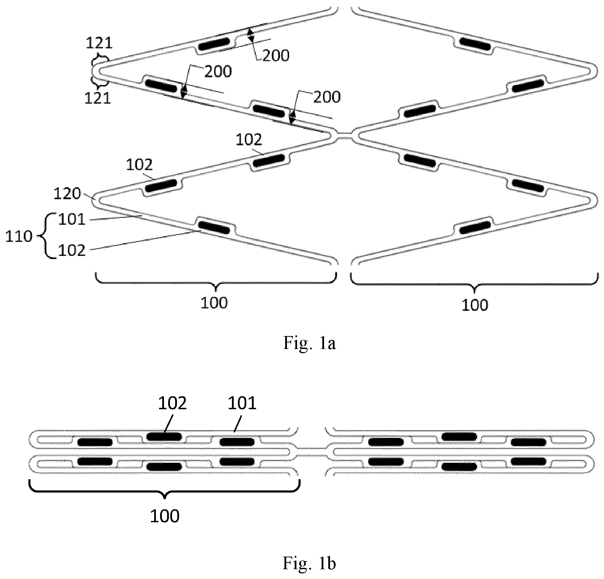

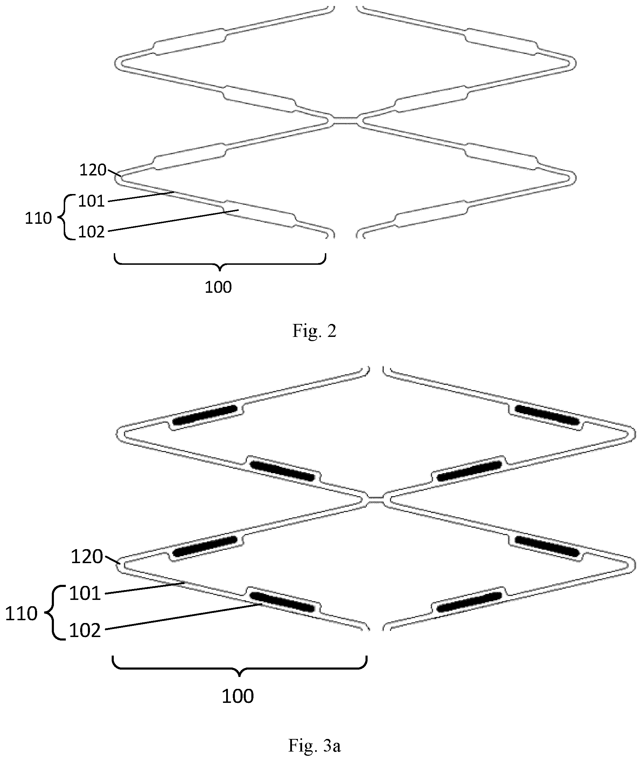

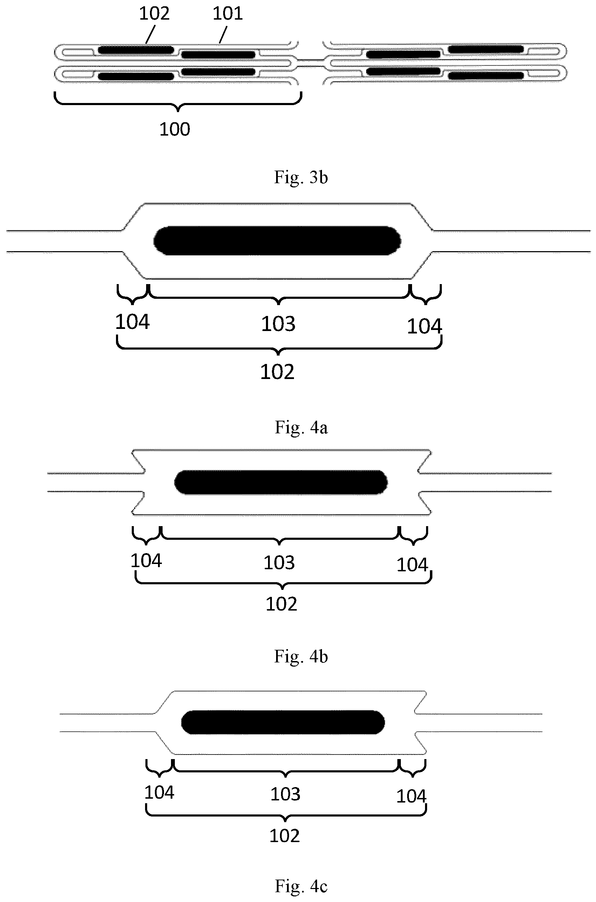

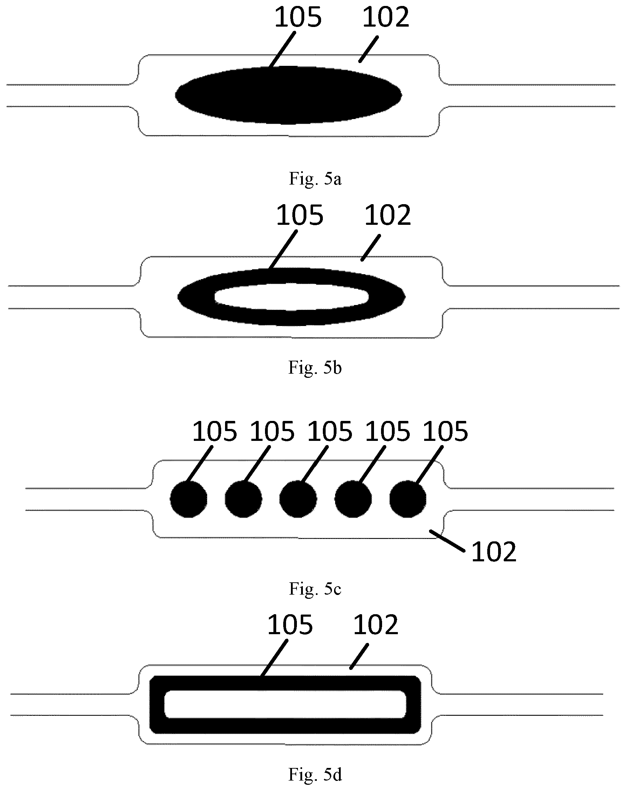

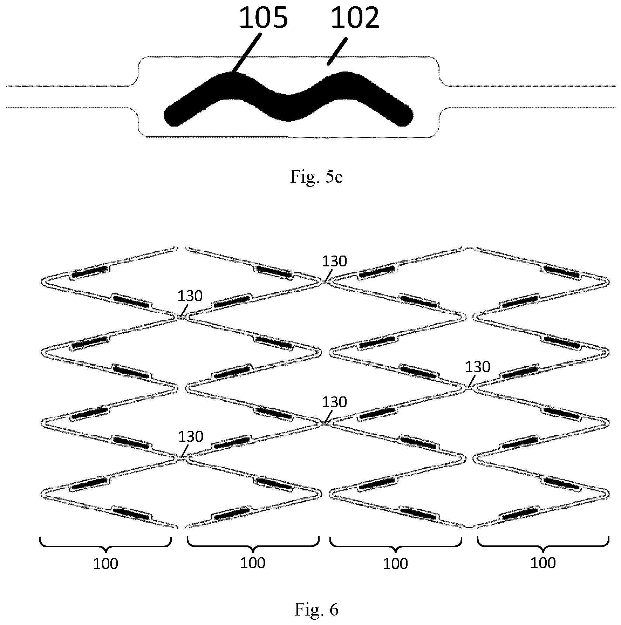

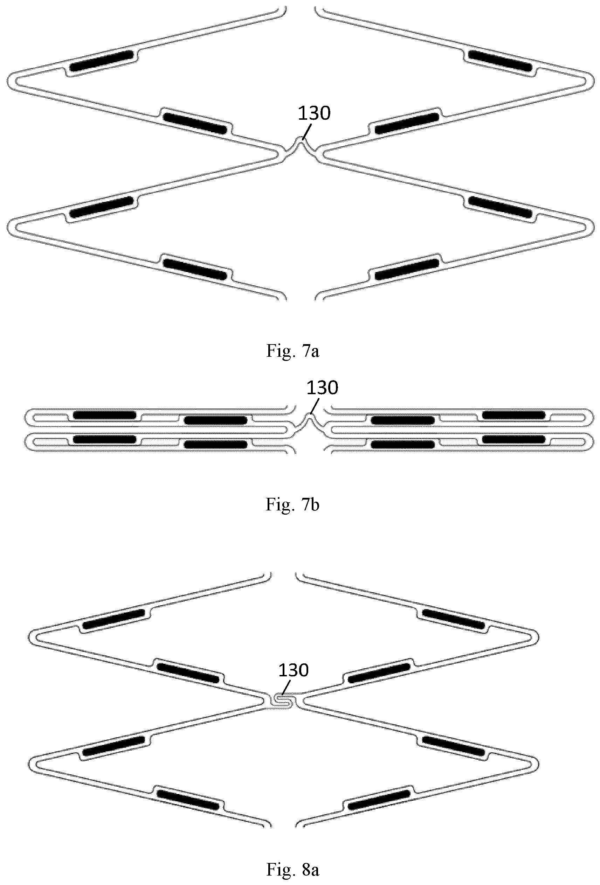

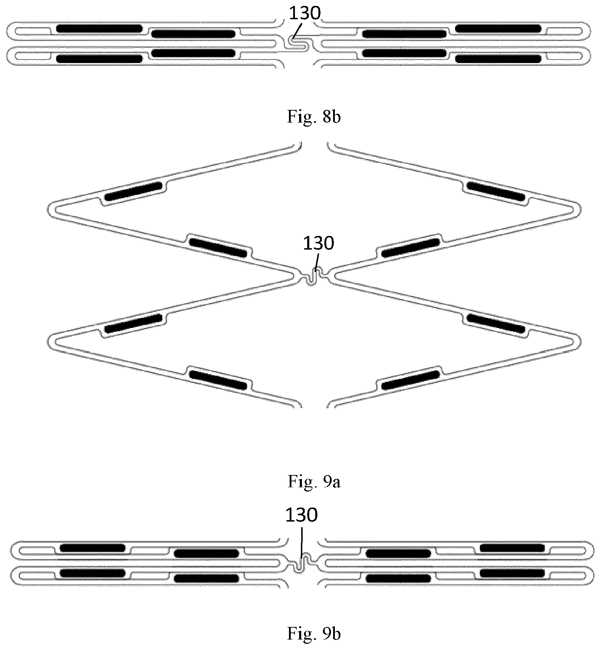

Reference will be made to Figs. 1a to 9b. Fig. 1a is a schematic partial view of a stent mesh according to an embodiment of the present invention. Fig. 1b is a schematic diagram showing the stent mesh of Fig. 1a in a collapsed configuration. Fig. 2 is a schematic partial view of a stent mesh according to another embodiment of the present invention. Fig. 3a is a schematic partial view of a stent mesh according to yet another embodiment of the present invention. Fig. 3b is a schematic diagram showing the stent mesh of Fig. 3a in a collapsed configuration. Fig. 4a is a schematic diagram showing a broadened section according to an embodiment of the present invention. Fig. 4b is a schematic diagram showing a broadened section according to another embodiment of the present invention. Fig. 4c is a schematic diagram showing a broadened section according to yet another embodiment of the present invention. Fig. 5a schematically illustrates a first optional implementation of a cavity pattern according to the present invention. Fig. 5b schematically illustrates a second optional implementation of a cavity pattern according to the present invention. Fig. 5c schematically illustrates a third optional implementation of a cavity pattern according to the present invention. Fig. 5d schematically illustrates a fourth optional implementation of a cavity pattern according to the present invention. Fig. 5e schematically illustrates a fifth optional implementation of a cavity pattern according to the present invention. Fig. 6 is a schematic partial view of a stent according to an embodiment of the present invention. Fig. 7a is a schematic partial view of a stent mesh according to the present invention, particularly showing an implementation of joints therein. Fig. 7b is a schematic diagram showing the stent mesh of Fig. 7a in a collapsed configuration. Fig. 8a is a schematic partial view of a stent mesh according to the present invention, particularly showing another implementation of joints therein. Fig. 8b is a schematic diagram showing the stent mesh of Fig. 8a in a collapsed configuration. Fig. 9a is a schematic partial view of a stent mesh according to the present invention, particularly showing yet another implementation of joints therein. Fig. 9b is a schematic diagram showing the stent mesh of Fig. 9a in a collapsed configuration.

As shown in Figs. 1a and 1b, in an embodiment, a stent is provided. The stent includes a plurality of stent meshes 100 each including plurality of stent struts 110, which are sequentially connected end to end circumferentially around the stent mesh 100, and a joint is formed at the connected ends of adjacent stent struts. The stent meshes 100 are configured to expand or collapse as a result of widening or narrowing of angles at the joints 120. Each stent strut 110 includes at least one main section 101 and at least one broadened section 102. The main section 101 has a width smaller than that of the broadened section 102. The broadened sections 102 of adjacent stent struts 110 are staggered along an axis of the stent mesh 100. In at least one of the stent struts, a total length of the at least one broadened section 102 accounts for 5% to 95% of a length of the stent strut 110. When the angles at the joints are in a range of 0° to 5°, metal coverage of the single stent mesh ranges from 30% to 99 %. The lengths are measured along an axis of the stent strut. In this embodiment, the widths are measured in directions perpendicular to axes of the objects being measured and tangential to an outer circumferential surface of the stent mesh 100. In Figs. 1a to 9b, the directions in which the widths are measured are perpendicular to the axes of the objects being measured and a line of sight of the viewer. In Fig. 1a, the width directions are illustrated and labeled as 200. Any other width or length as mentioned herein below is measured in a similar way.

Metal coverage of the stent refers to a ratio of a total outer surface area of its metal portions to a total area of a vessel segment that is covered by the stent. Specifically, it can be expressed as the formula: Metal Coverage = Ss/πDL × 100%, where Ss represents the outer surface area of the stent's metal portions, D is an outer diameter of the implanted stent segment, and L is a length of the implanted stent segment. It would be appreciated that the metal coverage depends on the structure of the stent and thus can be increased by appropriately modifying the structure of the stent, optionally in an expanded or collapsed configuration.

It would be appreciated that, in the stent shown in Figs. 1a and 1b, there are a plurality of stent meshes 100. What is shown in Figs. 1a and 1b is only a part of the stent mesh 100 (as is the case of Figs. 3a to 3b and Figs. 6 to 9b described below), and a whole stent mesh 100 may be constructed from multiple replicas of the portion shown in Figs. 1a and 1b, which may be connected together. In various embodiments, each single stent mesh 100 may include 8 to 24, preferably, 12 to 18, stent struts 110. Likewise, the stent includes a plurality of (e.g., 2 to 18) stent meshes 100. Each stent mesh 100 is a ringshaped structure made up of stent struts 110 which are connected end to end, and multiple such stent meshes 100 are connected axially to form the stent. When in an expanded configuration as shown in Fig. 1a, the stent meshes 100 can be used to support human tissue such as a blood vessel. When in a collapsed configuration as shown in Fig. 1b, the stent meshes 100 are suitable for passage through a limited space, such as a lumen of a blood vessel. The main sections 101 of the stent struts 110 allow the stent to overall occupy a smaller space, while the broadened sections 102 can ensure that metal coverage of the stent is large enough to enable desirable coverage of a plaque by the stent. The different widths of the broadened sections 102 and the main sections 101, as well as the staggered arrangement of the broadened sections 102, enable the stent to have desirable radial strength which can ensure that the stent is able to provide support at a stenotic site while not causing damage to a blood vessel.

Referring to Fig. 2, in some embodiments, the stent provided herein may not have cavities. However, this is undesirable according to an analysis of drug-loaded stents performed by the inventors. Most existing drug loading techniques based on a self-expanding stent involve coating a surface of the stent with a drug layer or with drug-containing film. There is a known self-expanding stent, which is surface-coated with a polymer film formed by spraying drug-containing polymer nanofibers onto the stent surface. There is another known self-expanding luminal stent which is entirely or partially coated with a film, with a drug being attached to the film. There is yet another known stent comprising a shape memory metal mesh with a coating film and a drug layer applied onto the film. All these techniques inevitably suffer from premature wear or peeling of the drug layer during crimping, loading and delivery of the self-expanding stent, and filling the drug into cavities formed in the stent is considered as a good solution to this problem. In fact, this design has found use in some stents for treating stenosis of extracranial arteries, such as coronary drug-eluting stents. Therefore, in some embodiments, cavities for containing a drug therein may be formed in the broadened sections, in order to increase drug loading capacity of the metal sections. This drug loading capacity can be achieved because: 1) the broadened sections 102 are wider and therefore satisfy the basic space requirements for drug loading; and 2) loading the drug in the cavity instead of coating it onto the stent surface circumvents the problem of wear or peeling of the drug during crimping, loading and delivery of the stent. This arrangement enables desirable space utilization and takes into account of both drug loading capacity and radial strength, allowing the stent to have a compact size in the collapsed configuration, which facilitates passage of the stent through a lesion site. Therefore, the stent can provide satisfactory ability to pass through a lesion site, desirable drug loading capacity and good radial strength.

With the above arrangement, the stent can have a radial dimension smaller than or equal to 0.0419 cm (0.0165 inches) when in the collapsed configuration, which enables the stent to be used with the smallest available delivery microcatheters. In all embodiments, the stent may have an outer diameter of 0.3 to 0.7 mm when in the collapsed configuration, which allows the stent to be used with microcatheters with an inner diameter in the range of 0.033 cm (0.013 inches) to 0.068 cm (0.027 inches).

The inventors have carried out tests for ratios of total length of the broadened section(s) 102 to length the stent strut 110. All the tests stents had an outer diameter of 0.53 mm in the collapsed configuration, a total length of 9.68 mm in the collapsed configuration, a thickness of 0.07 mm and a stent mesh count of 6. Starting with all these same initial dimensions in the collapsed configuration, the stents expanded to a radial dimension of 3 mm, when their radial strength and metal coverage were measured. The results are shown in Table 1.

**Table 1 Dependence of Radial Strength and Metal Coverage on Ratio of Broadened Sections**

| No. | ratio of total length of broadened section(s) to length of stent strut (%) | ratio of total length of broadened section(s) in adjacent stent struts to length of stent strut (%) | radial strength (kPa) | metal coverage (%) |
|---|---|---|---|---|
| 1 | 0 | 0 | 6.77 | 7.51 |
| 2 | 0 | 100 | 10.5 | 13.12 |
| 3 | 50 | 50 | 9 | 11.31 |
| 4 | 10 | 90 | 9.1 | 12.85 |
| 5 | 5 | 5 | 6.92 | 8.62 |
| 6 | 5 | 95 | 9.7 | 12.93 |
| 7 | 30 | 30 | 8.71 | 11.11 |
| 8 | 15 | 15 | 7.23 | 8.93 |
| 9 | 20 | 20 | 7.56 | 9.24 |
| 10 | 25 | 75 | 9.23 | 12.84 |

As can be seen from the data in Table 1, in comparison to the results of Test No. 1 serving as a control test, the stents with ratios of total length of the broadened section(s) 102 in one strut to length of the stent strut 110 ranging from 5% to 95% exhibit both desirable radial strength and sufficiently large metal coverage.

As can be also seen from the data in Table 1, the ratio of total length of the broadened section(s) 102 in one strut to length of the stent strut 110 is preferably 15% to 75%, more preferably 20% to 50%. In some specific embodiments, the ratio of total length of the broadened section(s) 102 in one strut to length of the stent strut 110 may be 5%, 10%, 15%, 20%, 25%, 30%, 35%, 40%, 45%, 50%, 55%, 60%, 65%, 70%, 75%, 80%, 85%, 90%, 95% or the like.

Additionally, the ratio of the total length of the broadened section(s) 102 in any adjacent two stent struts 110 to the length of the stent strut 110 does not exceed 100%. As also can be seen from the data in Table 1, a ratio of the total length of the broadened section(s) 102 in adjacent stent strut 110 to the length of stent strut 110 may remain the same or vary.

As also can be seen from the data in Table 1, the stents show radial strength of 1 kPa to 300 kPa.

In various embodiments, the length of the stent struts 110 may range from 1 mm to 2.5 mm. In one preferred embodiment, the length of the stent struts 110 may range from 1.2 mm to 1.5 mm. The stent struts 110 may be generally linear in shape, or may have one or more curved portions, but they extend overall in the lengthwise direction.

In various embodiments, the stent struts 110 may have a width of 20 mm to 150 mm. In various embodiments, the width of the main sections 101 may range from 30 µm to 50 µm. In various embodiments, the width of the broadened sections 102 may range from 90µm to 120 µm.

The metal coverage of the stent is determined by its structure and the angles at the joints 120. When the structure of the stent is same, the angles at the joints 120 are defined by adjacent stent struts 110 connected to the joint 120. When the angles at the joints 120 are 0°, the stent is the aforementioned collapsed configuration where the two stent struts 110 connected to any joint 120 define an angle of 0° therebetween, resulting in large metal coverage of the stent. When the angles at the joints 120 are 30° to 120°, the stent is the aforementioned expanded configuration where it shows reduced metal coverage. In the collapsed configuration, at a given total metal surface area of the stent and a given thickness of the struts therein, larger metal coverage means a smaller outer diameter of the stent, which allows the stent to be more easily advanced in a blood vessel and to be delivered to a more distant target site or a target site in a narrower blood vessel. In the expanded configuration, larger metal coverage allows the stent to more desirably cover a plaque at a lesion site, providing better outcomes. The design with alternating wide and narrow strut sections enables the stent to have effectively improved metal coverage while exhibiting satisfactory radial strength. Metal coverage of stent meshes 100 with different ratios of broadened sections at various angles is shown in Table 2.

**Table 2 Metal Coverage of Stent Meshes with Different Ratios of Broadened Sections at Various Angles**

| No. | ratio of total length of broadened section(s) to length of stent strut (%) | ratio of total length of broadened section(s) in adjacent stent strut to length of stent strut (%) | metal coverage of single stent mesh (%) | | | | | |
|---|---|---|---|---|---|---|---|---|
| | | | angle at 0° | angle at 5° | angle at 30° | angle at 60° | angle at 90° | angle at 120° |
| 1 | 5 | 5 | 38.44 | 33.86 | 8.81 | 4.78 | 4.16 | 4.03 |
| 2 | 30 | 30 | 53.50 | 47.12 | 11.18 | 6.65 | 5.79 | 5.61 |
| 3 | 50 | 50 | 94.39 | 83.14 | 11.88 | 11.74 | 10.21 | 9.89 |
| 4 | 10 | 90 | 96.45 | 84.95 | 12.02 | 11.99 | 10.43 | 10.11 |
| 5 | 5 | 95 | 96.14 | 84.67 | 12.06 | 11.96 | 10.40 | 10.08 |
| 6 | 0 | 100 | 95.82 | 84.39 | 12.89 | 11.92 | 10.37 | 10.04 |

As can be seen from the data in Table 2, when the ratio of total length of the broadened section(s) 102 in one strut to length of the stent strut 110 is in the range of 5% to 95%, and when the stent is in a configuration where the angles at the joints in the stent range from 0° to 5°, each single stent mesh 100 has metal coverage of 30% to 99%. As can be also seen from Table 2, in said configuration of the stent where the angles at the joints in the stent range from 0° to 5°, each single stent mesh 100 is preferred to have metal coverage of 80% to 99%.

As can be seen from the data in Table 2, the angles at the joints 120 may range from 0° to 120°. In some other embodiments, the angles at the joints 120 may range from 0° to 140°. When the angles at the joints 120 are 0° or almost 0°, the stent is in the collapsed configuration. This can be achieved by crimping the stent into a guide sheath before loaded into a microcatheter. The stent remains in the collapsed configuration after it is loaded into the microcatheter and during the subsequent delivery therein. Notably, it is not the case that the stent is in the collapsed configuration only when the angles at the joints 120 are 0°. Depending on an inner diameter of the microcatheter, the stent may be loaded therein with various angles at the joints 120. A larger inner diameter of the microcatheter will lead to larger angles at the joints 120. For example, the angles at the joints 120 may be 0° to 5° in the collapsed configuration of the stent. When the angles at the joints 120 lie between 30° and 120°, the stent comprises the expanded configuration. In some embodiments, when the stent expands to an outer diameter of 2 mm to 3 mm, the angles at the joints 120 are in the range of 30° to 90°. It is noted that the stent does not necessarily fully expand in the expanded configuration. Rather, it may expand to a predetermined outer diameter. For example, when placed in a tube with an inner diameter of 2 mm to 3 mm, the stent may expand to an outer diameter of 2 mm to 3 mm in the expanded configuration. At this time, the angles at the joints 120 are greater than those in the collapsed configuration. After being taken out of the tube or transferred into a tube with a greater inner diameter, the stent may further expand, leading to an increase in the angles at the joints 120.

As can be seen from the data in Table 2, when the angles at the joints are in the range of 5° to 30°, each single stent mesh shows metal coverage of 5% to 90%. When the angles at the joints are in the range of 30° to 90°, each single stent mesh shows metal coverage of 4% to 15%. As can be also seen from the data in Table 2, the metal coverage of each single stent mesh 100 is preferred to be 8% to 15% when the angles at the joints in the stent is in the range of 30° to 90°. When the angles at the joints are in the range of 90° to 140°, each single stent mesh shows metal coverage of 3% to 12%.

Apart from the metal coverage of the individual stent meshes 100, overall metal coverage of the stent also depends on linking struts 130 between the stent meshes. The longer the linking struts 130, the more distant the stent meshes 100 are spaced apart from each other. Due to the presence of the linking struts 130, the overall metal coverage of the stent is smaller than the metal coverage of the individual stent meshes 100. When the linking struts 130 are equally long and of the same structure, the greater the number of linking struts between the stent meshes 100, the greater the metal coverage of the stent. In addition, the metal coverage of the stent also depends on the structure of the linking struts. In the embodiments provided herein, when the angles at the joints are in the range of 0° to 5°, the overall metal coverage of the stent is 20% to 60%, preferably 35 to 60%; when the angles at the joints are in the range of 5° to 30°, the overall metal coverage of the stent is 5% to 45%; when the angles at the joints are in the range of 30° to 90°, the overall metal coverage of the stent is 3% to 15%, preferably 8% to 15%; and when the angles at the joints are in the range of 90° to 140°, the overall metal coverage of the stent is 2% to 15%.

In various embodiments, each stent strut 110 may include 1 to 10 broadened sections 102. Preferably, each stent strut 110 includes one broadened section 102. This enables good space utilization and ensures large metal coverage and/or a sufficient drug load. Moreover, each stent strut 110 may include 1 to 11 main sections 101. Preferably, each stent strut 110 includes 2 main sections 101. The main sections 101 and the broadened sections 102 are alternately arranged. A width ratio of the main sections 101 to the broadened sections 102 may range from 1/4 to 2/3. Preferably, the width ratio is 1/3, which enables good space utilization. Adjacent stent struts 110 may include the same number or different numbers of broadened sections 102. Each stent strut 110 may include one or more broadened sections 102. Referring to Fig. 1a, in one embodiment, one stent strut 110 includes three main sections 101 and two broadened sections 102 that are alternately arranged. Each broadened section is located between two main sections. Moreover, in the same stent mesh 100, an adjacent stent strut includes two main sections 101 and one broadened section 102 located between the main sections. With continued reference to Fig. 2, in a preferred embodiment, each stent strut 110 in a stent mesh 100 includes two main sections 101 and one broadened section 102 located between the main sections.

Each stent mesh 100 further includes bend sections 121 for connecting the stent struts 110. In various embodiments, a ratio of width of the main sections 101 to width of the bends 121 may range from 1/2 to 3/4. Preferably, the ratio is 3/5, which can prevent breakage at the joints 120 while enabling the stent to have low strength.

In various embodiments, for each stent strut 110, a ratio of length of stent strut 110 to length of the shortest main section 101 therein may be greater than or equal to 10/3, preferably 6/1.

In various embodiments, a ratio of length of the stent struts 110 to length of the bend sections 121 may be greater than or equal to 8. Preferably, this ratio is 12, which can prevent breakage at the joints 120 while enabling the stent to have low strength.

Preferably, when the angles at the joints 120 are minimized, in each stent strut 110, each broadened section 102 does not overlap with any main section 101 in an adjacent stent strut 110 connected to the same joint 120. During design, backward inference may be used to calculate a dimension of the broadened sections 102 from a radial distance between the main sections 101 in adjacent stent struts 110 at the minimized angles, under the premise of ensuring overall structural strength and a designed service lifespan. This can additionally enhance space utilization and enable the stent to have an even smaller overall size. In some embodiments, the main sections 101 in different pairs of adjacent stent struts 110 connected to respective joints 120 may be spaced by different radial distances when the angles are minimized. In this case, the widths of the broadened sections 102 in the stent struts 110 may be individually designed in order to achieve good space utilization. Alternately, all the broadened sections 102 may be designed with the same width, according to the smallest radial distance of all the pairs of adjacent stent struts 110 at the minimized angles. This represents a low-cost design approach.

It would be appreciated that the above-discussed embodiments are preferred, but in some special cases, when the angles at the joints 120 are minimized, in each pair of adjacent stent struts 110 connected to the same joint 120, the radial distance between their main sections 101 may also be smaller than the widths of protrusion of the broadened sections 102 relative to the main sections 101 on one side. This can also achieve good space utilization.

As shown in Fig. 2, in one embodiment, the broadened sections 102 have margins of the same width beyond the main sections 101 at opposite sides of the stent struts along the lengthwise direction thereof. Center axes of the broadened sections 102 coincide with center axes of the stent struts 110, optionally with offsets within a manufacturing tolerance range.

In some other embodiments, the broadened sections 102 may have margins of different widths beyond the main sections 101 at opposite sides of the stent struts along the lengthwise direction thereof. For example, as shown in Figs. 3a and 3b, in one embodiment, the broadened sections 102 are flush with the main sections 101 at one side of the stent strut along the lengthwise direction thereof. Preferably, in the expanded configuration of the stent, each pair of adjacent stent struts 110 connected to the same joint 120 forms a V-shaped structure, wherein the sides of the adjacent stent struts 110, at which the broadened sections 102 are flush with the main sections 101, are both located at inner side or outer side of the V-shaped structure.

In other embodiments, the sides of the adjacent stent struts 110, at which the broadened sections 102 are flush with the main sections 101 may be located at different sides of the V-shaped structure. In other words, in the expanded configuration of the stent, each pair of adjacent stent struts 110 connected to the same joint 120 forms a V-shaped structure, wherein the sides of the adjacent stent struts 110, at which the broadened sections 102 are flush with the main sections 101, are not both located at inner or outer sides of the V-shaped structure. That is, for adjacent stent struts, the side of one stent strut 110, at which the broadened sections 102 are flush with the main sections 101, is located at the inner side of the V-shaped structure, while the side of the adjacent stent strut 110, at which the broadened sections 102 are flush with the main sections 101, is located at the outer side of the V-shaped structure.

Referring to Figs. 4a to 4c, each broadened section 102 includes a main segment 103 and transition segments 104 each joining the main segment 103 to one main section 101. The transition segments 104 may comprise any of various shapes. For example, in one embodiment, the transition segments 104 are each tapered from the main segment 103 toward the main section 101 (see Fig. 4a). In an alternative embodiment, the transition segments 104 each include two outwardly open transition notches on opposite sides of the main section 101. The transition segments 104 are as wide as the main segment 103, and the openings of the transition notches are tapered from the main section 101 toward the main segment 103 (see Fig. 4b). In another alternative embodiment, out of the transition segments 104 of all the broadened sections 102, at least some have at least one of the above-discussed shapes (i.e., any of the examples shown in Figs. 4a, 4b and 4c). It would be appreciated that the transition segments 104 may also be configured otherwise. In some other embodiments, for example, in the embodiment shown in Fig. 3a, each broadened section 102 may only include the main segment 103 and, in this case, the main segment 103 is directly connected to the main sections 101.

Since the broadened sections have a relatively large surface area, cavities can be formed therein, in which a drug or radiopaque substance may be filled to increase the stent's drug loading capacity or radiopacity. Referring to Figs. 5a to 5e, the broadened sections 102 may include cavities 105, the cavity 105 is formed by cavity pattern(s). The cavity pattern is at least one of a solid pattern, a hollow pattern, a continuous pattern and a discontinuous pattern. The cavity may have a shape composed of at least one of those of a cylinder, a cuboid and a triangular prism. Each cavity may have a transverse cross-sectional shape composed of at least one of a circular shape, an elongate shape, a polygonal shape, a corrugated shape, an annular shape and an irregular shape. Each cavity may have a longitudinal cross-sectional shape composed of at least one of an arcuate shape, a quadrilateral shape and a triangular shape. Notably, the transverse cross-sectional shape refers to the shape of a cross-section taken parallel to an outer surface the broadened section 102 in which the cavity is formed, and the longitudinal cross-sectional shape refers to the shape of a cross-section taken perpendicular to both the outer surface and a lengthwise direction of the broadened section 102. The cavities 105 are configured for a drug or radiopaque substance to be filled therein. In some embodiments, the cavities 105 may be through slots extending through the stent strut 110 across its entire thickness. In some other embodiments, the cavities 105 may be blind holes extending through a partial thickness of the stent struts 110 and terminating somewhere therein. It would be appreciated that Figs. 4a to 4c are meant to particularly exemplify and illustrate the transition segments 104, and Figs. 5a to 5e are meant to particularly exemplify and illustrate the cavities 105. It is in no way intended that when the transition segments 104 are configured as shown in any of Figs. 4a to 4c, the cavities 105 have to be shaped as shown in the same figure, or that when the cavities are configured as shown in any of Figs. 5a to 5e, the transition segments 104 have to be shape as shown in the same figure. These implementations may be combined in any combination.

In various embodiments, a ratio of a maximum width of the cavity pattern to the width of the broadened sections 102 may be in the range of 1/3 to 4/5. Preferably, this ratio is 1/2, because it can enable good space utilization and ensure a sufficient drug load. The width of the cavity pattern is measured in the same direction as the width of the broadened sections 102 is measured.

Referring to Fig. 1a, in one embodiment, the stent includes at least two axially connected stent meshes 100. The stent may include 4 to 18 stent meshes 100, preferably 6 to 10 stent meshes 100. When the stent expands to an outer diameter of 2 mm to 3 mm, each of the illustrated stent meshes may have a length of 1 mm to 1.8 mm. The stent meshes may be connected to each other in any suitable way. In a preferred embodiment, referring to Fig. 6, the stent includes linking struts 130, and the joints 120 in adjacent stent meshes 100 are aligned and interconnected by the linking struts 130. In the embodiment shown in Fig. 6, the joints 120 in adjacent stent meshes 100 are aligned, and the two stent meshes 100 are in mirrored symmetry with each other. Additionally, referring to Fig. 6, the linking struts 130 may connect pairs of joints 120 interposed between non-connected pairs circumferentially around the stent. This arrangement can facilitate the widening or narrowing of the angles at the joints and enhance space utilization. It would be appreciated that, in alternative embodiments, the joints 120 may be not aligned and the linking struts may not be arranged at the joints 120. Moreover, when the joints 120 are aligned and connected by the linking struts 130, the connections may be made in another way than as discussed above. For example, all pairs of aligned joints 120 may be each connected with a linking strut 130. Alternately, the pairs of aligned joints 120 may be connected with the linking struts 130 at an interval of two non-connected pairs circumferentially around the stent. Still other arrangements are possible.

In various embodiments, a ratio of width of the main sections 101 to width of the linking struts 130 may be in the range of 1/2 to 1. This ratio is preferred to be 1/2, in order to satisfactorily prevent breakage of the linking struts 130.

Referring to Figs. 1a to 1b and 7a to 9b, the linking struts 130 may have a shape composed of at least one of a linear shape, a corrugated shape, a serrated shape, a circular shape, an annular shape, a "Ω"-like shape and an "S"-like shape. The linking struts 130 are preferred to be non-linear in shape, for example, corrugated, "Ω"-shaped or "S"-shaped, because these shapes can facilitate overall collapse and expansion of the stent. It would be appreciated that various implementations of the linking struts 130 may be combined with those of the above-discussed cavities 105, transition segments 104 and broadened sections 102 in any combination.

In other embodiments, cavities may also be formed in the linking struts 130. However, this is not favorable from the point of view of overall structural reliability.

Referring to Figs. 1 to 3b and 7a to 9b, in some embodiments, when the angles at the joints 120 are minimized, in any of stent mesh 100, the broadened sections 102 in adjacent stent struts 110 may be spaced apart by certain distances along the axis of the stent mesh 100 (see Figs. 1b, 7b and 9b). In a more preferred embodiment, when the angles at the joints 120 are minimized, in each stent mesh 100, the broadened sections 102 in adjacent stent struts 110 may come into snug contact along the axis of the stent mesh 100 (see Figs. 3b and 8b).

In some embodiments, the stent may be formed of a cobalt-based alloy, a magnesium alloy, a nickel-titanium alloy, stainless steel or the like, or a combination thereof.

There is also provided herein a drug-loaded stent differing from the stent as defined above mainly in including cavities 105, in which a drug or radiopaque substance is filled.

For details of other features of the drug-loaded stent, reference can be made to the above description of this specification in connection with the stent.

This invention provides a stent including at least one stent mesh 100 each including a plurality of stent struts 110, which are sequentially connected end to end circumferentially, and a joint is formed at the connected ends of adjacent stent struts. The stent mesh 100 is configured to expand or collapse as a result of widening or narrowing of angles at the joints 120. Each stent strut 110 includes at least one main section 101 and at least one broadened section 102 that are alternately arranged. The main section 101 has a width smaller than that of the broadened section 102. In each stent mesh 100, the broadened sections 102 of adjacent stent struts 110 are staggered along an axis of the stent mesh 100. This invention also provides a drug-loaded stent of a similar structure. The different widths of the broadened sections 102 and the main sections 101, as well as the staggered arrangement of the broadened sections 102, enable reasonable space utilization and achieve a good tradeoff between metal coverage and radial strength. In this way, the stent is allowed to have a compact size when in a collapsed configuration, which facilitates passage of the stent through a lesion site. Therefore, the problem of failing to provide both good sufficient metal coverage and desirable radial strength associated with conventional stents is overcome.

The description presented above is merely that of some preferred embodiments of the present invention and is not intended to limit the scope thereof in any sense.

## Claims

1. A stent, comprising at least one stent mesh (100), wherein each stent mesh (100) comprises a plurality of stent struts (110) sequentially connected circumferentially around the stent mesh (100), wherein the plurality of the stent struts (110) are sequentially connected end to end, and a joint (120) is formed at the connected ends of adjacent stent struts (110), and wherein the stent mesh (100) is configured to expand or collapse as a result of widening or narrowing of angles at the joints (120),
wherein the stent strut (110) comprises at least one main section (101) and at least one broadened section (102) that are alternately arranged, wherein the main section (101) has a width smaller than a width of the broadened section (102), wherein in each stent mesh (100), the broadened sections (102) of adjacent stent struts (110) are staggered along an axis of the stent mesh (100), wherein in at least one of the stent struts (110), a total length of the at least one broadened section (102) accounts for 5% to 95% of a length of the stent strut (110);
**characterized in that** when the angle at each joint (120) is in a range of 0° to 5°, a metal coverage of the stent mesh (100) ranges from 30% to 99%, and when the angle at each joint (120) is in a range of 5° to 30°, the metal coverage of the stent mesh (100) ranges from 5% to 90%, and
**in that** in at least one of the stent struts (110), the broadened section (102) is flush with the main section (101) at one side of the stent strut (110) along a lengthwise direction thereof, and wherein in an expanded configuration of the stent, the adjacent stent struts (110) connected to a same joint (120) form a V-shaped structure, wherein sides of the adjacent stent struts (110), at which the main section (101) is flush with the broadened section (102), are simultaneously located at an inner side or an outer side of the V-shaped structure.

2. The stent according to claim 1, wherein in at least one of the stent struts (110), a total length of the at least one broadened section (102) accounts for 15% to 75% of a length of the stent strut (110).

3. The stent according to claim 1, wherein in at least one of the stent struts (110), a total length of the at least one broadened section (102) accounts for 20% to 50% of a length of the stent strut (110).

4. The stent according to claim 1, wherein in adjacent two stent struts (110), a ratio of a total length of the broadened sections (102) of the adjacent two stent struts (110) to a length of a single stent strut (110) does not exceed 100%; or
wherein the angle at each joint (120) ranges from 0° to 140°; or
wherein the stent has a radial strength in a range of 1 kPa to 300 kPa; or
wherein the stent strut (110) comprises two main sections (101) and one broadened section (102) that is located between the two main sections (101); or
wherein the stent mesh (100) comprises 8 to 24 stent struts (110); or
wherein when the angles at the joints (120) are minimized, in each adjacent stent struts (110) connected to a same joint (120), the broadened section (102) of one of the stent struts (110) does not overlap with the main section (101) of the adjacent stent strut (110).

5. The stent according to claim 1, wherein when the angle at each joint (120) is in a range of 30° to 90°, the metal coverage of the stent mesh (100) ranges from 4% to 15%, optionally wherein when the angle at each joint (120) is in a range of 30° to 90°, the metal coverage of the stent mesh (100) ranges from 8% to 15%.

6. The stent according to claim 1, wherein when the angle at each joint (120) is in a range of 90° to 140°, the metal coverage of the stent mesh (100) ranges from 3% to 12%.

7. The stent according to claim 1, wherein when the angle at each joint (120) is in a range of 0° to 5°, the metal coverage of the stent ranges from 20% to 60%.

8. The stent according to claim 1, wherein when the angle at each joint (120) is in a range of 5° to 30°, the metal coverage of the stent ranges from 5% to 45%.

9. The stent according to claim 1, wherein when the angle at each joint (120) is in a range of 30° to 90°, the metal coverage of the stent ranges from 3% to 15%.

10. The stent according to claim 1, wherein when the angle at each joint (120) is in a range of 90° to 140°, the metal coverage of the stent ranges from 2% to 15%.

11. The stent according to claim 1, wherein at least one of the broadened sections (102) comprises a cavity (105), optionally wherein: the broadened section (102) comprises 1 to 10 cavities (105); or
a longitudinal cross-sectional shape of the cavity (105) comprises at least one of an arcuate shape, a quadrilateral shape and a triangular shape; or
a transverse cross-sectional shape of each cavity (105) comprises at least one of a circular shape, an elongate shape, a polygonal shape, a corrugated shape, an annular shape and an irregular shape; or
the cavity (105) is configured for a drug or radiopaque substance to be filled therein.

12. The stent according to any one of claims 1 to 11, wherein the stent comprises at least two stent meshes (100) that are axially connected, optionally wherein the stent comprises at least one linking strut (130), and wherein the linking strut (130) connects the joints (120) in adjacent stent meshes (100), optionally wherein a shape of the linking strut (130) comprises at least one of a linear shape, a corrugated shape, a serrated shape, a circular shape, an annular shape, a "Ω"-like shape and an "S"-like shape.

13. A drug-loaded stent, comprising the stent of claim 1, wherein the stent comprises at least one stent mesh (100), wherein each stent mesh (100) comprises a plurality of stent struts (110) sequentially connected circumferentially around the stent mesh (100), wherein the plurality of the stent struts (110) sequentially connected end to end, and a joint (120) is formed at the connected ends of adjacent stent struts (110), and wherein the stent mesh (100) is configured to expand or collapse as a result of widening or narrowing of angles at the joints (120),
wherein the stent strut (110) comprises at least one main section (101) and at least one broadened section (102) that are alternately arranged, wherein the main section (101) has a width smaller than a width of the broadened section (102), wherein in each stent mesh (100), the broadened sections (102) of adjacent stent struts (110) are staggered along an axis of the stent mesh (100), and wherein the broadened section (102) comprises a cavity (105) configured for a drug or a radiopaque substance to be filled therein;
wherein in at least one stent strut (110), a total length of the at least one broadened section (102) accounts for 5% to 95% of a length of the stent strut (110), and wherein when the angle at each joint (120) is in a range of 0° to 5°, a metal coverage of the stent mesh (100) ranges from 30% to 99%.

## Patentansprüche

1. Stent, der mindestens ein Stentgeflecht (100) umfasst, wobei jedes Stentgeflecht (100) eine Vielzahl von Stentstreben (110) umfasst, die nacheinander in Umfangrichtung um das Stentgeflecht (100) herum verbunden sind, wobei die Vielzahl der Stentstreben (110) nacheinander Ende an Ende verbunden ist und ein Gelenk (120) an den verbundenen Enden von angrenzenden Stentstreben (110) gebildet ist, und wobei das Stentgeflecht (100) so konfiguriert ist, dass es sich als Ergebnis von Erweitern oder Verengen von Winkeln an den Gelenken (120) ausdehnt oder zusammenzieht,
wobei die Stentstrebe (110) mindestens einen Hauptabschnitt (101) und mindestens einen verbreiterten Abschnitt (102) umfasst, die abwechselnd angeordnet sind, wobei der Hauptabschnitt (101) eine Breite aufweist, die kleiner ist als eine Breite des verbreiterten Abschnitts (102), wobei in jedem Stentgeflecht (100) die verbreiterten Abschnitte (102) von angrenzenden Stentstreben (110) entlang einer Achse des Stentgeflechts (100) gestaffelt sind, wobei bei mindestens einer der Stentstreben (110) eine Gesamtlänge des mindestens einen verbreiterten Abschnitts (102) 5 % bis 95 % einer Länge der Stentstrebe (110) ausmacht;
**dadurch gekennzeichnet, dass**, wenn der Winkel an jedem Gelenk (120) in einem Bereich von 0° bis 5° liegt, eine Metallbedeckung des Stentgeflechts (100) von 30 % bis 99 % reicht, und wenn der Winkel an jedem Gelenk (120) in einem Bereich von 5° bis 30° liegt, die Metallabdeckung des Stentgeflechts (100) von 5% bis 90 % reicht, und
dadurch, dass bei mindestens einer der Stentstreben (110) der verbreiterte Abschnitt (102) mit dem Hauptabschnitt (101) an einer Seite der Stentstrebe (110) entlang einer Längsrichtung davon bündig ist, und wobei in einer ausgedehnten Konfiguration des Stents die angrenzenden Stentstreben (110), die mit einem gleichen Gelenk (120) verbunden sind, eine V-förmige Struktur bilden, wobei Seiten der angrenzenden Stentstreben (110), an denen der Hauptabschnitt (101) mit dem verbreiterten Abschnitt (102) bündig ist, gleichzeitig auf einer Innenseite oder einer Außenseite der V-förmigen Struktur gelegen sind.

2. Stent nach Anspruch 1, wobei bei mindestens einer der Stentstreben (110) eine Gesamtlänge des mindestens einen verbreiterten Abschnitts (102) 15 % bis 75 % der Länge der Stentstrebe (110) ausmacht.

3. Stent nach Anspruch 1, wobei bei mindestens einer der Stentstreben (110) eine Gesamtlänge des mindestens einen verbreiterten Abschnitts (102) 20 bis 50 % der Länge der Stentstrebe (110) ausmacht.

4. Stent nach Anspruch 1, wobei bei zwei angrenzenden Stentstreben (110) ein Verhältnis einer Gesamtlänge der verbreiterten Abschnitte (102) der zwei angrenzenden Stentstreben (110) zu einer Länge einer einzelnen Stentstrebe (110) nicht 100 % übersteigt; oder
wobei der Winkel an jedem Gelenk (120) von 0° bis 140° reicht; oder
wobei der Stent eine radiale Festigkeit in einem Bereich von 1 kPa bis 300 kPa aufweist; oder
wobei die Stentstrebe (110) zwei Hauptabschnitte (101) und einen verbreiterten Abschnitt (102) umfasst, der zwischen den zwei Hauptabschnitten (101) gelegen ist; oder
wobei das Stentgeflecht (100) 8 bis 24 Stentstreben (110) umfasst; oder
wobei, wenn die Winkel an den Gelenken (120) minimiert sind, bei jeder angrenzenden Stentstreben (110), die mit einem gleichen Gelenk (120) verbunden sind, der verbreiterte Abschnitt (102) von einer der Stentstreben (110) nicht mit dem Hauptabschnitt (101) der angrenzenden Stentstrebe (110) überlappt.

5. Stent nach Anspruch 1, wobei, wenn der Winkel an jedem Gelenk (120) in einem Bereich von 30° und 90° liegt, die Metallabdeckung des Stentgeflechts (100) von 4 % bis 15 % reicht, optional wobei, wenn der Winkel an jedem Gelenk (120) in einem Bereich von 30° bis 90° liegt, die Metallabdeckung des Stentgeflechts (100) von 8 % bis 15 % reicht.

6. Stent nach Anspruch 1, wobei, wenn der Winkel an jedem Gelenk (120) in einem Bereich von 90° bis 140° liegt, die Metallabdeckung des Stentsgeflechts (100) von 3 % bis 12 % reicht.

7. Stent nach Anspruch 1, wobei, wenn der Winkel an jedem Gelenk (120) in einem Bereich von 0° bis 5° liegt, die Metallabdeckung des Stents von 20 % bis 60 % reicht.

8. Stent nach Anspruch 1, wobei, wenn der Winkel an jedem Gelenk (120) in einem Bereich von 5° bis 30° liegt, die Metallabdeckung des Stents von 5 % bis 45 % reicht.

9. Stent nach Anspruch 1, wobei, wenn der Winkel an jedem Gelenk (120) in einem Bereich von 30° bis 90° liegt, die Metallabdeckung des Stents von 3 % bis 15 % reicht.

10. Stent nach Anspruch 1, wobei, wenn der Winkel an jedem Gelenk (120) in einem Bereich von 90° bis 140° liegt, die Metallabdeckung des Stents von 2 % bis 15 % reicht.

11. Stent nach Anspruch 1, wobei mindestens einer der verbreiterten Abschnitte (102) einen Hohlraum (105) umfasst, optional wobei: der verbreiterte Abschnitt (102) 1 bis 10 Hohlräume (105) umfasst; oder
eine Längsschnittsform des Hohlraums (105) mindestens eines umfasst von einer bogenförmigen Form, einer vierseitigen Form oder einer dreieckigen Form; oder
eine Querschnittsform jedes Hohlraums (105) mindestens eines umfasst von einer kreisförmigen Form, einer länglichen Form, einer polygonalen Form, einer gewellten Form, einer ringförmigen Form oder einer unregelmäßigen Form; oder
der Hohlraum (105) so konfiguriert ist, dass darin ein Arzneimittel oder eine strahlenundurchlässige Substanz eingefüllt werden kann.

12. Stent nach einem der Ansprüche 1 bis 11, wobei der Stent mindestens zwei Stentgeflechte (100) umfasst, die axial verbunden sind, optional wobei der Stent mindestens eine Verbindungsstrebe (130) umfasst, und wobei die Verbindungsstrebe (130) die Gelenke (120) in angrenzenden Stentgefechten (100) verbindet, optional wobei eine Form der Verbindungsstrebe (130) mindestens eines umfasst von einer linearen Form, einer gewellten Form, einer gezahnten Form, einer kreisförmigen Form, einer ringförmigen Form, einer "Ω"-ähnlichen Form oder einer "S"-ähnlichen Form.

13. Mit Arzneimitteln beladener Stent, der den Stent nach Anspruch 1 umfasst, wobei der Stent mindestens ein Stentgeflecht (100) umfasst, wobei jedes Stentgeflecht (100) eine Vielzahl von Stentstreben (110) umfasst, die nacheinander in Umfangrichtung um das Stentgeflecht (100) herum verbunden ist, wobei die Vielzahl der Stentstreben (110) nacheinander Ende an Ende verbunden ist und ein Gelenk (120) an den verbundenen Enden von angrenzenden Stentstreben (110) gebildet ist, und wobei das Stentgeflecht (100) so konfiguriert ist, dass es sich als Ergebnis von Erweitern oder Verengen von Winkeln an den Gelenken (120) ausdehnt oder zusammenzieht,
wobei die Stentstrebe (110) mindestens einen Hauptabschnitt (101) und mindestens einen verbreiterten Abschnitt (102) umfasst, die abwechselnd angeordnet sind, wobei der Hauptabschnitt (101) eine Breite aufweist, die kleiner ist als eine Breite des verbreiterten Abschnitts (102), wobei in jedem Stentgeflecht (100) die verbreiterten Abschnitte (102) von angrenzenden Stentstreben (110) entlang einer Achse des Stentgeflechts (100) gestaffelt sind, und wobei der verbreiterte Abschnitt (102) einen Hohlraum (105) umfasst, der so konfiguriert ist, dass darin ein Arzneimittel oder eine strahlenundurchlässige Substanz eingefüllt werden kann,
wobei bei mindestens einer Stentstrebe (110) eine Gesamtlänge des mindestens einen verbreiterten Abschnitts (102) 5 % bis 95 % einer Länge der Stentstrebe (110) ausmacht, und wobei, wenn der Winkel an jedem Gelenk (120) in einem Bereich von 0° bis 5° liegt, eine Metallbedeckung des Stentgeflechts (100) von 30 % bis 99 % reicht.

## Revendications

1. Stent, comprenant au moins une maille de stent (100), dans lequel chaque maille de stent (100) comprend une pluralité de montants de stent (110) reliés séquentiellement circonférentiellement autour de la maille de stent (100), dans lequel la pluralité de montants de stent (110) sont reliés séquentiellement bout à bout, et une articulation (120) est formée aux extrémités reliées des montants de stent adjacents (110), et dans lequel la maille de stent (100) est configurée pour se dilater ou se contracter en raison de l'élargissement ou du rétrécissement des angles au niveau des articulations (120),
dans lequel le montant de stent (110) comprend au moins une section principale (101) et au moins une section élargie (102) agencées alternativement, dans lequel la section principale (101) présente une largeur inférieure à celle de la section élargie (102), dans lequel, dans chaque maille du stent (100), les sections élargies (102) des montants de stent adjacents (110) sont décalées le long d'un axe de la maille de stent (100), dans lequel, dans au moins un des montants de stent (110), la longueur totale de l'au moins une section élargie (102) représente 5 % à 95 % de la longueur du montant de stent (110) ;
**caractérisé en ce que**, lorsque l'angle au niveau de chaque articulation (120) est compris entre 0° et 5°, une couverture métallique de la maille de stent (100) varie de 30 % à 99 %, et lorsque l'angle au niveau de chaque articulation (120) est compris entre 5° et 30°, la couverture métallique de la maille de stent (100) varie de 5 % à 90 %, et
dans lequel, dans au moins un des montants de stent (110), la section élargie (102) est alignée avec la section principale (101) d'un côté du montant de stent (110) dans le sens de sa longueur, et dans lequel, dans une configuration déployée du stent, les montants adjacents du stent (110) reliés à une même articulation (120) forment une structure en forme de V, dans lequel les côtés des montants adjacents du stent (110), au niveau desquels la section principale (101) est alignée avec la section élargie (102), sont simultanément situés d'un côté intérieur ou d'un côté extérieur de la structure en forme de V.

2. Stent selon la revendication 1, dans lequel, dans au moins un des montants du stent (110), la longueur totale de l'au moins une section élargie (102) représente de 15 % à 75 % de la longueur du montant du stent (110).

3. Stent selon la revendication 1, dans lequel, dans au moins un des montants du stent (110), la longueur totale de l'au moins une section élargie (102) représente 20 % à 50 % de la longueur du montant du stent (110).

4. Stent selon la revendication 1, dans lequel, dans deux montants de stent adjacents (110), le rapport entre la longueur totale des sections élargies (102) des deux montants de stent adjacents (110) et la longueur d'un seul montant de stent (110) ne dépasse pas 100 % ; ou
dans lequel l'angle au niveau de chaque articulation (120) varie de 0° à 140° ; ou
dans lequel le stent présente une résistance radiale dans une plage comprise entre 1 kPa et 300 kPa ; ou
dans lequel le montant du stent (110) comprend deux sections principales (101) et une section élargie (102) située entre les deux sections principales (101) ; ou
dans lequel la maille du stent (100) comprend de 8 à 24 montants de stent (110) ; ou
dans lequel, lorsque les angles au niveau des articulations (120) sont minimisés, dans chaque montant de stent adjacent (110) relié à une même articulation (120), la section élargie (102) de l'un des montants de stent (110) ne chevauche pas la section principale (101) du montant de stent adjacent (110).

5. Stent selon la revendication 1, dans lequel, lorsque l'angle au niveau de chaque articulation (120) est dans une plage comprise entre 30° et 90°, la couverture métallique de la maille du stent (100) varie de 4 % à 15 %, éventuellement, lorsque l'angle au niveau de chaque articulation (120) est dans une plage comprise entre 30° et 90°, la couverture métallique de la maille de stent (100) varie de 8 % à 15 %.

6. Stent selon la revendication 1, dans lequel, lorsque l'angle au niveau de chaque articulation (120) est dans une plage comprise entre 90° et 140°, la couverture métallique de la maille du stent (100) varie de 3 % à 12 %.

7. Stent selon la revendication 1, dans lequel, lorsque l'angle au niveau de chaque articulation (120) est dans une plage comprise entre 0° et 5°, la couverture métallique du stent varie de 20 % à 60 %.

8. Stent selon la revendication 1, dans lequel, lorsque l'angle au niveau de chaque articulation (120) est dans une plage comprise entre 5° et 30°, la couverture métallique du stent varie de 5 % à 45 %.

9. Stent selon la revendication 1, dans lequel, lorsque l'angle au niveau de chaque articulation (120) est dans une plage comprise entre 30° et 90°, la couverture métallique du stent varie de 3 % à 15 %.

10. Stent selon la revendication 1, dans lequel, lorsque l'angle au niveau de chaque articulation (120) est dans une plage comprise entre 90° et 140°, la couverture métallique du stent varie de 2 % à 15 %.

11. Stent selon la revendication 1, dans lequel au moins une des sections élargies (102) comprend une cavité (105), et, en option dans lequel : la section élargie (102) comprend de 1 à 10 cavités (105) ; ou
la forme en coupe longitudinale de la cavité (105) comprend au moins une forme parmi une forme arquée, une forme quadrilatérale et une forme triangulaire ; ou
la forme en coupe transversale de chaque cavité (105) comprend au moins une forme parmi une forme circulaire, allongée, polygonale, ondulée, annulaire ou irrégulière ; ou
la cavité (105) est configurée pour y être remplie d'un médicament ou d'une substance radio-opaque.

12. Stent selon l'une quelconque des revendications 1 à 11, dans lequel le stent comprend au moins deux mailles de stent (100) qui sont reliées axialement, dans lequel le stent comprend éventuellement au moins un montant de liaison (130), et dans lequel le montant de liaison (130) relie les articulations (120) dans les mailles de stent adjacentes (100), éventuellement dans lequel une forme du montant de liaison (130) comprend au moins une forme parmi une forme linéaire, une forme ondulée, une forme dentelée, une forme circulaire, une forme annulaire, une forme en « Ω » et une forme en « S ».

13. Stent chargé de médicament, comprenant le stent selon la revendication 1, dans lequel le stent comprend au moins une maille de stent (100), dans lequel chaque maille de stent (100) comprend une pluralité de montants de stent (110) reliés séquentiellement circonférentiellement autour de la maille de stent (100), dans lequel la pluralité des montants de stent (110) sont reliés séquentiellement bout à bout, et une articulation (120) est formée aux extrémités reliées des montants de stent adjacents (110), et dans lequel la maille de stent (100) est configurée pour se dilater ou se contracter en raison de l'élargissement ou du rétrécissement des angles au niveau des articulations (120),
dans lequel le montant du stent (110) comprend au moins une section principale (101) et au moins une section élargie (102) agencées alternativement, dans lequel la section principale (101) présente une largeur inférieure à celle de la section élargie (102), dans lequel, dans chaque maille du stent (100), les sections élargies (102) des montants adjacents du stent (110) sont décalées le long d'un axe de la maille du stent (100), et dans lequel la section élargie (102) comprend une cavité (105) configurée pour être remplie d'un médicament ou d'une substance radio-opaque ;
dans lequel, dans au moins une maille de stent (110), la longueur totale de l'au moins une section élargie (102) représente de 5 % à 95 % de la longueur du montant de stent (110), et lorsque l'angle au niveau de chaque articulation (120) est compris entre 0° et 5°, le taux de couverture métallique de la maille de stent (100) varie de 30 % à 99 %.
